(19) Europäisches Patentamt
European Patent Office
Office européen des brevets

(11) **EP 4 261 276 A1**

(12) **EUROPEAN PATENT APPLICATION**
published in accordance with Art. 153(4) EPC


(43) Date of publication:
**18.10.2023 Bulletin 2023/42**

(21) Application number: **21823457.3**

(22) Date of filing: **04.05.2021**

(51) International Patent Classification (IPC):
***C12N 5/077*** *(2010.01)* ***C07K 14/78*** *(2006.01)*
***C07K 14/495*** *(2006.01)* ***A61K 35/33*** *(2015.01)*
***A61P 21/00*** *(2006.01)*

(52) Cooperative Patent Classification (CPC):
**A61K 35/33; A61P 21/00; C07K 14/495; C07K 14/78; C12N 5/06**

(86) International application number:
**PCT/KR2021/005591**

(87) International publication number:
**WO 2022/114412 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2020 KR 20200162290**



(71) Applicant: **Tego Science Inc.**
**Gangseo-gu, Seoul 07794 (KR)**

(72) Inventors:
- **JEON, Saewha**
**Seoul 07794 (KR)**
- **HAN, Jikhyon**
**Seoul 07794 (KR)**
- **KIM, Yun Hee**
**Seoul 07794 (KR)**
- **LEE, Ji-Hye**
**Seoul 07794 (KR)**

(74) Representative: **Plasseraud IP**
**66, rue de la Chaussée d'Antin**
**75440 Paris Cedex 09 (FR)**


(54) **SKIN-DERIVED FIBROBLASTS EXHIBITING TENDON REGENERATION EFFECT AND USE THEREOF**


(57) The present invention relates to human skin-derived fibroblasts having a tendon regeneration effect and a pharmaceutical composition for regenerating and treating a tendon, and the allogeneic skin-derived fibroblasts may have similar cell morphology to tenocytes, have a high expression level of vimentin, which is a fibroblast marker, express a large amount of extracellular matrix proteins, TGF-β, and increase collagen synthesis by acting on an ERK signaling mechanism, thereby effectively regenerating an injured tendon.


Fig 1

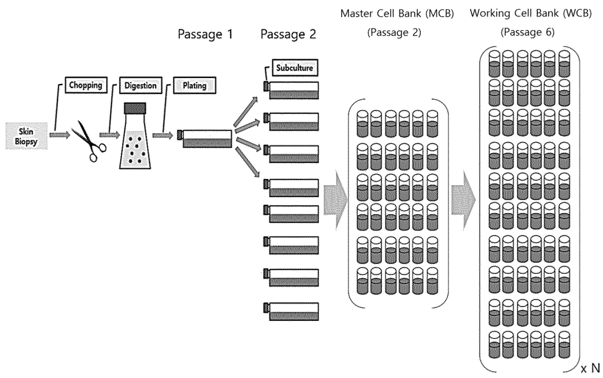


EP 4 261 276 A1

## Description

[Technical Field]

The present invention relates to a skin-derived fibroblast exhibiting a tendon regeneration effect and a pharmaceutical composition for regenerating and treating a tendon, which includes the same as an active ingredient.

[Background Art]

A tendon is connective tissue that connects muscle to bone to transmit a force generated in the muscle to the bone, and induces joint movement, and consists of tenocytes and an extracellular matrix (ECM) such as collagen. Tendon cells are tendon-specific fibroblasts that play a role in the production and maintenance of the tendon matrix. Tendon injury due to trauma is common in exercise, work and daily life, and tendon inflammation or a partial rupture due to degenerative changes caused by aging may occur even with minor trauma.

A rotator cuff tear refers to a partial or total rupture of tendon caused by such as tendon aging of four muscles that wrap around the shoulder blades and help the arms move freely, such as the supraspinatus, infraspinatus, teres minor and subscapularis. Rotator cuff tears account for approximately 70% of shoulder lesions, and the incidence is increasing with aging and the increase in sports population. Most treatments up to now are surgical suture, but the rate of rerupture after suturing is high so that the development of alternative treatment is necessary.

While there is autologous cell therapy using tenocytes as a commercially available cell-like therapeutic until now, there is a disadvantage in that it should be accompanied by surgery to collect a patient's normal tendon tissue in order to obtain raw cells for cell therapy.

However, if fibroblasts can be applied as a therapeutic, since fibroblasts may be easily collected by a relatively non-invasive method, and may be prepared in a read-made type ensuring quality standards to be immediately used when needed, it is possible to solve the problem of a cell therapeutic using the autologous tenocytes. Therefore, there is a need to develop a therapeutic for tendon injuries using fibroblasts, in addition to allogeneic tenocytes.

[Disclosure]

[Technical Problem]

To solve the above problems, the present inventors attempted to develop a treatment for tendon injuries using fibroblasts, and confirmed that fibroblasts which are derived from human skin, and increased in expression of one or more selected from the group consisting of vimentin, collagen I, collagen III, collagen V, fibronectin and elastin compared to tenocytes have a tendon regeneration effect, and thus the present invention was completed.

[Technical Solution]

To solve the above problems, one aspect of the present invention provides fibroblasts (skin-derived fibroblasts) which are derived from human skin, and increased in expression of one or more selected from the group consisting of vimentin, collagen I, collagen III, collagen V, fibronectin and elastin compared to tenocytes and have a tendon regeneration effect.

The "fibroblasts" used herein refer to a mesenchymal cell having high diversity, found in various tissues, and according to the US NIH database Medical Subject Headings (MeSH), the fibroblasts are connective tissue cells which secrete an extracellular matrix rich in collagen and other macromolecules.

The "tendon" used herein refers to connective tissue that connects muscle to bone and transmits the force generated by the muscle to the bone, thereby causing joint movement. Type I collagen accounts for 85 to 95% of the tendon weight, type III collagen accounts for approximately 5% of the tendon weight, and proteoglycan accounts for approximately 5% of the tendon weight. In addition, fibronectin or elastin provides a solid cell scaffold in tissue.

The inventors used fibroblasts that are able to be obtained from autologous or allogeneic cells by a relatively non-invasive method to solve problems of a cell therapeutic using autologous tenocytes. Specifically, normal skin tissue isolated from a donor was minced, and only fibroblasts were isolated by lysing a cell matrix. The donor may be a person evaluated as suitable for construction of a cell bank through suitability assessment, and may not be limited to a specific donor. Accordingly, the skin-derived fibroblasts of the present invention may be autologous or allogeneic. The allogeneic fibroblasts refer to fibroblasts originating from a different person.

The fibroblasts isolated from a donor begun primary culture at 37 °C in 10% $CO_2$, and unlike normal cell culture, 10% $CO_2$ was used in cell culture to maintain the pH of a cell culture medium at 7.4. When the cells in a culture dish were filled to a pre-confluent density of approximately 70 to 80%, subculture was carried out, and a master cell bank was established at passage 2, and a working cell bank was established at passage 6.

Therefore, according to one embodiment of the present invention, the skin-derived fibroblasts were subcultured two passages or more, and preferably 6 passages or more, and subcultured in a medium with pH 6.5 to 8.0.

In addition, according to one embodiment of the present invention, the skin-derived fibroblasts have a population doubling level per passage of 2.5 to 5.0, which is maintained at a relatively predetermined level even when subculture is continued. On the other hand, human tenocytes have a considerably reduced population doubling level when subculture is continued (FIG. 2). Here, one passage refers to cells cultured for approximately 6 days at pre-confluent density after cell seeding in a culture dish.

According to one embodiment of the present invention, the skin-derived fibroblasts may be increased in expression of cytokines and signaling materials, which are reported to be involved in formation of the ECM and promotion of tissue regeneration. Specifically, compared to tenocytes, the secretion of transforming growth factor-β (TGF-β) increases, and the expression of phosphorylated extracellular-signal regulated kinase 1/2 (p-ERK 1/2) involved in extracellular-signal regulated kinase (ERK) signaling and the expression of collagen I also increase.

In addition, since the skin-derived fibroblasts of the present invention have a cell survival rate of approximately 70% or more even when thawed after long-term storage, and the ECM, collagen, is detected at 0.10 μg or more per the number of unit cells of $10^6$, and therefore, cell quality was also remarkably excellent.

Therefore, another aspect of the present invention provides a pharmaceutical composition for alleviating or treating a tendon disease, which includes the fibroblasts having a tendon regeneration effect as an active ingredient.

According to one embodiment of the present invention, the tendon diseases may be selected from a rotator cuff tear, an Achilles tendon disease, tendinitis, a tendon injury and tendon detachment, and preferably, a rotator cuff tear.

The inventors confirmed that, as a result of introducing fibroblasts having a tendon regeneration effect to a rotator cuff tear animal model, tendon tissue is generated to a level similar to that of the normal group without a rotator cuff tear (FIG. 8).

In addition, to confirm the mechanism of tendon regeneration, as a result of treating tenocytes with a lysate of fibroblasts having a tendon regeneration effect, it was confirmed that the expression of p-ERK 1/2 involved in ERK signaling is significantly increased, and the expression of collagen I is also increased (FIG. 9).

The pharmaceutical composition for alleviating or treating a tendon disease may further include an adjuvant such as a polymer carrier that can improve efficacy, for example, hyaluronic acid, alginate or fibrin. The polymer carrier should be biocompatible and serve to assist cell delivery and do not affect a cell survival rate and improve physical properties, and therefore may improve efficacy.

In addition, the pharmaceutical composition for alleviating or treating a tendon disease may further include a cell cryoprotectant, dimethyl sulfoxide (DMSO), and may be included at 2 to 15% of a cell freezing medium. DMSO prevents dehydration by changing the concentration of a non-permeable extracellular solution during ice formation at the time of cell freezing.

In addition, the pharmaceutical composition for alleviating or treating a tendon disease may further include a pharmaceutically acceptable carrier in addition to the active ingredient. The pharmaceutically acceptable carrier included in the composition is conventionally used in the production of a preparation, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil, but the present invention is not limited thereto.

The pharmaceutical composition for alleviating or treating a tendon disease is preferably administered parenterally. For parenteral administration, the pharmaceutical composition may be administered subcutaneously, intramuscularly, intraperitoneally, intradermally or locally, and directly injected into an injured site.

A suitable dosage of the pharmaceutical composition for alleviating or treating a tendon disease is in a range of 100 to100,000,000 ($10^2$ to $10^8$) cells/kg based on an adult. The term "pharmaceutically effective amount" refers to an amount sufficient to prevent or treat a tendon injury.

The composition may be prepared using a pharmaceutically acceptable carrier and/or excipient according to a method that can be easily performed by those of ordinary skill in the art, and may be prepared in a unit dose form or by being put into multidose containers. In addition, the composition may be administered as a separate therapeutic agent or in combination with another therapeutic agent, and may be administered sequentially or simultaneously with a conventional therapeutic agent. In addition, the composition may be additionally administered once or as needed, and may be used in combination with a surgical operation such as suturing or administered alone.

The pharmaceutical composition for alleviating or treating a tendon disease, which includes the fibroblasts having a tendon regeneration effect as an active ingredient may be provided in the form of a cell therapeutic agent. The "cell therapeutic agent" refers to drugs for the purpose of treatment, diagnosis and prevention through a series of actions such as *in vitro* proliferating and selecting living autologous, allogeneic or heterologous cells, or as another method, changing biological characteristics of cells to restore the tissue and function of cells.

In addition, the present invention provides a method of use in alleviating or treating a tendon disease, which includes administering the fibroblasts having a tendon regeneration effect of claim 1 or the pharmaceutical composition for alleviating or treating a tendon disease of claim 8 into a subject in need of treatment. The type of tendon disease, a dosage of the pharmaceutical composition or fibroblasts, and an administration method are the same as described in the pharmaceutical composition for alleviating or treating a tendon disease.

[Advantageous Effects]

Allogeneic skin-derived fibroblasts having a tendon regeneration effect according to one embodiment of the present invention have similar cell morphology to tenocytes, have a high expression level of vimentin, which is a fibroblast marker, express a large amount of extracellular matrix proteins, and increase collagen synthesis by acting on TGF-β and ERK signaling mechanisms, thereby efficiently regenerating an injured tendon.

[Description of Drawings]

FIG. 1 is a schematic diagram illustrating the construction of a human-derived allogeneic skin fibroblast bank with skin-derived fibroblasts according to one embodiment of the present invention.

FIG. 2 shows the result of confirming a population doubling level (PDL) according to subculture of human-derived tenocytes (A) and skin-derived fibroblasts (B).

FIG. 3 shows the result of observing *in vitro* cultured tenocytes and skin-derived fibroblasts of master and working cell banks using a phase contrast microscope.

FIG. 4 shows the result of observing the expression levels of vimentin in *in vitro* cultured tenocytes and skin-derived fibroblasts of a working cell bank.

FIG. 5 shows the result of observing the expression levels of keratin (K14) and pan-cytokeratin, which are keratinocyte markers, in skin-derived fibroblasts of a working cell bank.

FIG. 6 shows the result of observing the proportions of vimentin-expressing cells in skin-derived fibroblasts of a working cell bank.

FIG. 7 shows the results of observing the expression levels of an extracellular matrix protein in *in vitro* cultured tenocytes and skin-derived fibroblasts of a working cell bank using a fluorescence microscope (A) and western blotting (B).

FIG. 8 is the histological analysis results showing the degrees of recovery of tendon tissue after skin-derived fibroblasts are injected into rotator cuff tear animal models.

FIG. 9 shows the results confirming (A) a TGF-β level secreted from skin-derived fibroblasts, and (B) the change in expression of signaling molecules after tenocytes are treated with a cell lysate of skin-derived fibroblasts.

FIG. 10 shows the results of confirming the change in expression of signaling molecules after tenocytes are treated with a cell lysate of skin-derived fibroblasts at the gene level.

[Modes of the Invention]

Hereinafter, one or more embodiments will be described in further detail with reference to examples. However, these examples are merely provided to describe one or more embodiments, and the scope of the present invention is not limited to the following examples.

**Example 1: Culture of skin-derived fibroblasts**

Normal skin tissue isolated from a donor was minced and disrupted to isolate only fibroblasts. The isolated fibroblasts were subjected to primary culture in a Dulbecco's Modified Eagle's medium (DMEM)/F12 (Invitrogen, USA) supplemented with 10% fetal bovine serum (FBS), and as culture conditions, 37 °C and a 10% $CO_2$ wet environment were maintained. A master cell bank (MCB) was constructed at passage 2 after subculture at intervals of approximately 5 to 6 days. Afterward, following additional subculture at intervals of approximately 5 to 6 days, a working cell bank (WCB) was secured in large quantities at passage 6 (FIG. 1). The subculture of cells was performed by separating cells through trypsin-EDTA when the cell confluence reached approximately 70 to 80% in a culture medium. In most *in vitro* cell cultures, 5% $CO_2$ was used as a gas composition, but in the present invention, 10% $CO_2$ was used to accurately maintain the pH of the medium at 7.4.

The cell banks were frozen and stored as follows. First, cells at passages 2 to 10 and a freezing medium containing approximately 10% dimethyl sulfoxide (DMSO), which is a cryoprotectant for cells, were mixed. Afterward, 1 to $10\times10^6$ cells in a suspended state were dispensed in a glass ampoule, and were frozen while gradually dropping the temperature with isopropanol, and finally stored for a long time in a freezer at -15 °C or less or in liquid nitrogen.

Hereinafter, the skin-derived fibroblasts obtained in the example are described as "skin-derived fibroblasts."

**Example 2: Characterization of skin-derived fibroblasts**

**2-1. Proliferation and morphological characteristics of skin-derived fibroblasts**

The skin-derived fibroblasts obtained in Example 1 were cultured until passages 2 to 10 under the same culture condition, and a cell number in initial plating and a total cell number after collection of cells were counted to calculate a population doubling time. As a result, the population doubling time averaged 30 hours from passage 2 to passage 10, and similar values were obtained for two different media.

In addition, as a result of confirming a population doubling level (PDL) according to the following Equation 1, for the skin-derived fibroblasts obtained in Example 1, the PDL was calculated at 2.7 to 4.5 per passage (FIG. 2, lower graph; N=3, mean±SD). The result contrasts with tenocytes having a short cell lifespan during *in vitro* culture (FIG. 2, upper graph; N=3, mean±SD), and is one of the important advantages in application of fibroblasts as a cell therapeutic agent.

[Equation 1]

$$PDL = (\log(NH) - \log(NI))/\log 2$$

NH: Finally obtained cell number; NI: Initial plated cell number

**[0036]** In addition, as a result of confirming the morphology of skin-derived fibroblasts in the master and working cell banks manufactured in Example 1, it was confirmed that the skin-derived fibroblasts of the present invention maintain original spindle-shaped fibroblasts, similar to tenocytes (FIG. 3).

**2-2. Confirmation of vimentin expression**

The cultured tenocytes and skin-derived fibroblasts were treated with a primary antibody against a fibroblast marker protein, vimentin, and a green fluorescent material (FITC)-conjugated secondary antibody, and then observed by fluorescent microscopy. The cell nucleus was stained with 4,6-diamidino-2-phenylindole (DAPI).

As a result, it was confirmed that the skin-derived fibroblasts of the working cell bank show unique characteristics of fibroblasts, and express vimentin at a level similar to or higher than that of tenocytes (FIG. 4).

**2-3. Confirmation of homogeneity**

To confirm whether other cells are mixed and homogeneity in the skin-derived fibroblasts of the working cell bank, cells were stained with keratin (K14), which is a keratinocyte marker, and a pan-cytokeratin antibody to perform observation through fluorescence microscopy and flow cytometry. Cell nuclei were stained with DAPI.

As a result, no keratinocyte marker was detected in qualitative observation such as fluorescence microscopy (FIG. 5). In addition, in quantitative analysis such as flow cytometry, cells classified as cells expressing vimentin, which is a fibroblast marker, were detected at 98.5% on average, showing that most of the skin-derived fibroblasts constituting the working cell bank show fibroblast characteristics (FIG. 6).

**2-4. Confirmation of extracellular matrix protein**

In the skin-derived fibroblasts of the working cell bank, the expression level of a representative extracellular matrix, collagen, was confirmed. After the skin-derived fibroblasts were treated with a primary antibody against the extracellular matrix protein and a green fluorescent material (FITC)-conjugated secondary antibody, the cells were observed through fluorescence microscopy.

It was shown that the expression levels of collagen I, III and IV, fibronectin and elastin are very high in the skin-derived fibroblasts (FIG. 7).

**Example 3: Confirmation of quality of skin-derived fibroblasts**

The skin-derived fibroblasts of the cell banks, stored in cryopreservation, were thawed, and then cultured again to confirm their quality. As a result, it was confirmed that the thawing yield and cell survival rate of the skin-derived fibroblasts cultured again are excellent, indicating that cell quality was maintained regardless of the freezing period (Table 1).

[Table 1]

| Batch | Quality analysis of working cell bank | | | |
|---|---|---|---|---|
| | Freezing period (day) | Thawing yield (%) | Cell survival rate (%) | Amount of collagen per $10^6$ cells ($\mu$g) |
| 1 | 45 | 100 | 71 | 0.14 |
| 2 | 136 | 88.7 | 77 | 0.33 |
| 3 | 1830 | 98.5 | 72 | 0.32 |

In addition, as a result of conducting testing for detecting an adventitious virus and the test for identifying residual microorganisms, which meets the approval criteria for cell therapy products of the Ministry of Food and Drug Safety, it was confirmed that no foreign viruses and microorganisms remained in the cell banks.

**Experimental Example 1: Confirmation of therapeutic efficacy for rotator cuff tear**

**1-1. Preclinical efficacy**

The therapeutic efficacy for the skin-derived fibroblasts of the working cell bank for a rotator cuff tear was identified as follows.

Bilateral supraspinatus tears were performed on the shoulders of 20-week-old New Zealand white rabbits, and after 6 weeks, saline was injected into a control group, and $10^7$ skin-derived fibroblasts were injected into the experimental group and then sutured. Following suture, after 12 weeks had elapsed, it was confirmed that there were no abnormalities in a rabbit's weight and no other abnormal findings, and the rabbit was sacrificed to remove a shoulder tendon, and a paraffin block was prepared by decalcification. The paraffin block was stained with Masson's trichrome, and then histologically analyzed.

As a result of analysis, compared to the saline-injected control, in the experimental group injected with skin-derived fibroblasts, tendon tissue was aligned to a similar level to a normal group without a rotator cuff tear, and dense tendon tissue arrangement was observed (FIG. 8). In FIG. 8, the blue-stained part is collagen, the red-stained part is muscle, cytoplasm and keratin.

**1-2. Confirmation of mechanism of therapeutic efficacy**

To confirm the mechanism of the therapeutic efficacy for a rotator cuff tear of skin-derived fibroblasts according to one embodiment of the present invention, cytokines and signaling materials reported to be involved in formation of an extracellular matrix and promotion of tissue regeneration were analyzed.

When human-derived tenocytes and skin-derived fibroblasts of a working cell bank were cultured and transforming growth factor-$\beta$ (TGF-$\beta$) levels were quantified through enzyme-linked immunosorbent assay (ELISA), the skin-derived fibroblasts (HF) showed a higher secretion level of 692 pg of TGF-$\beta$ per $1 \times 10^6$ cells than the tenocytes (HT) (FIG. 9A).

In addition, 24 hours after the tenocytes were treated with a cell lysate of the skin-derived fibroblasts, cell signaling molecules involved in signaling were identified by western blotting. As a result, compared to a control, in an experimental group (HF lysate) treated with the lysate of the skin-derived fibroblasts, it was shown that the expression of phosphorylated extracellular-signal regulated kinase 1/2 (p-ERK 1/2) is significantly increased, and collagen I expression is also increased (FIG. 9B). In addition, at the gene level, it was confirmed that the expression of cell signal molecules is increased (FIG. 10).

As a result of the experimental example, it shows that the skin-derived fibroblasts of the present invention increase collagen I expression by ERK signaling, and thus exhibits a tendon regeneration effect (FIGS. 9 and 10).

## Claims

1. Fibroblasts having a tendon regeneration effect, which are derived from skin, and increased in expression of one or more proteins selected from the group consisting of vimentin, collagen I, collagen III, collagen V, fibronectin and elastin, compared to that of tenocytes.

2. The fibroblasts of claim 1, which are autologous or allogeneic.

3. The fibroblasts of claim 1, which are subcultured 2 or more passages.

4. The fibroblasts of claim 3, which are subcultured one or more passages in a medium with pH of 6.5 to 8.0.

5. The fibroblasts of claim 1, which have a population doubling level of 2.5 to 5.0 per passage.

6. The fibroblasts of claim 1, which are increased in transforming growth factor-β (TGF-β) compared to tenocytes.

7. The fibroblasts of claim 1, which activate an extracellular-signal regulated kinase (ERK) signaling pathway.

8. A pharmaceutical composition for alleviating or treating a tendon disease, comprising the fibroblasts having a tendon regeneration effect of claim 1 as an active ingredient.

9. The composition of claim 8, wherein the tendon disease is selected from the group consisting of a rotator cuff tear, an Achilles tendon disease, tendinitis, a tendon injury and tendon detachment.

10. The composition of claim 8, further comprising a polymer carrier selected from the group consisting of hyaluronic acid, alginate and fibrin.

11. A method of use in alleviating or treating a tendon disease, comprising:
administering the fibroblasts having a tendon regeneration effect of claim 1 or the pharmaceutical composition for alleviating or treating a tendon disease of claim 8 into a subject in need of treatment.

[FIGURES]

Fig 1

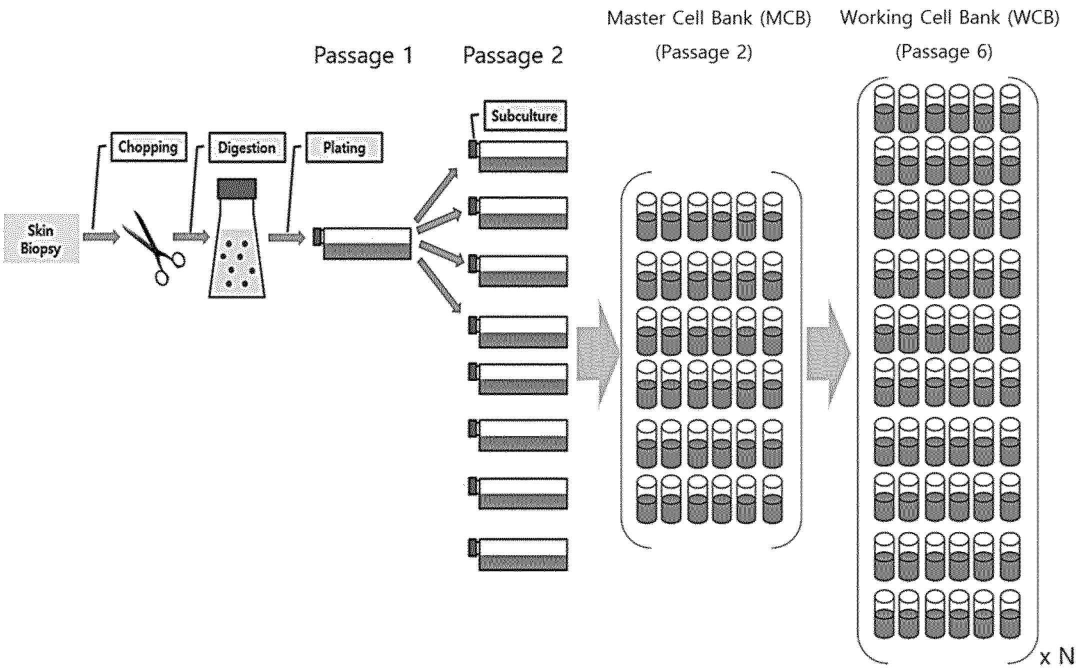

Fig 2

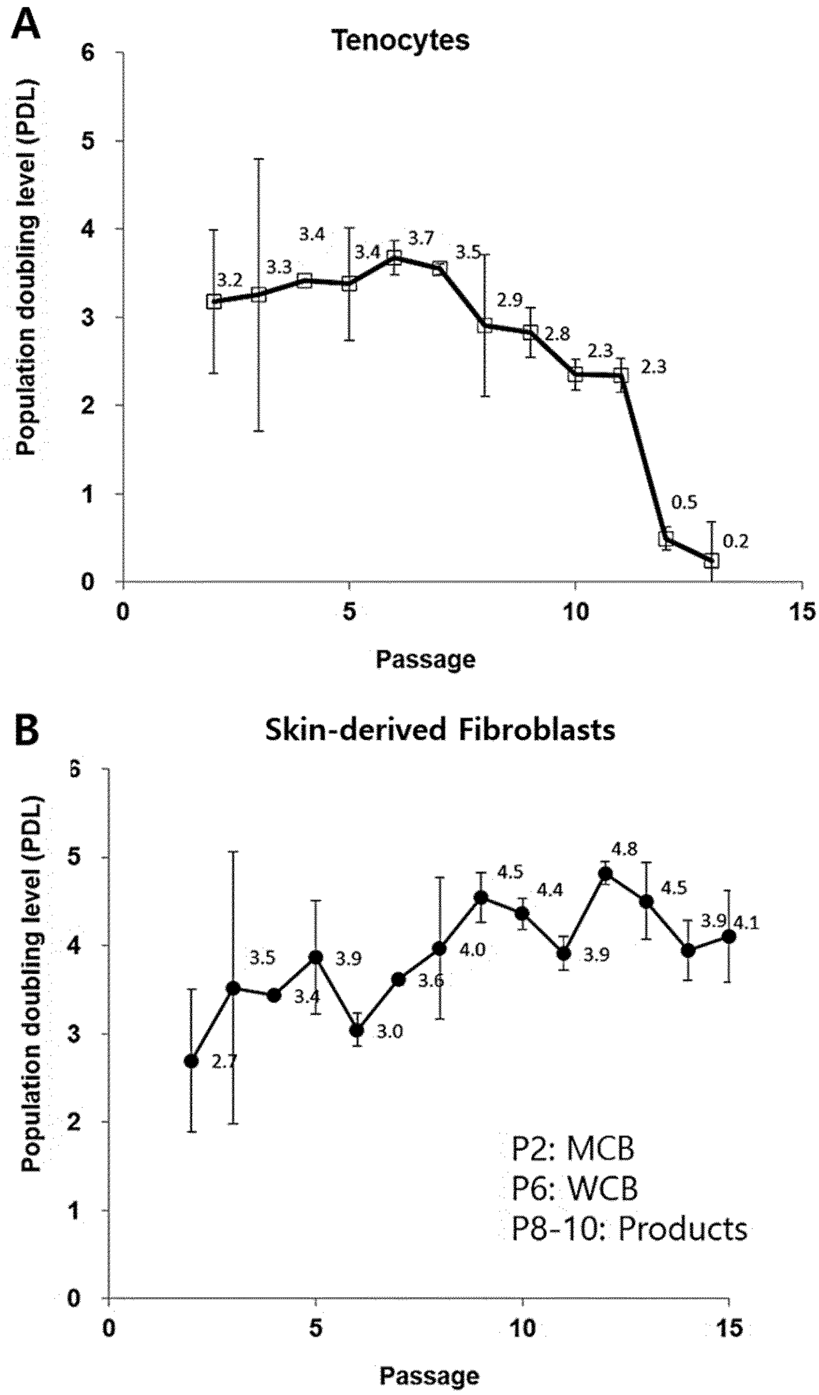
A
Tenocytes
Population doubling level (PDL)
Passage
B
Skin-derived Fibroblasts
Population doubling level (PDL)
Passage
P2: MCB
P6: WCB
P8-10: Products

Fig 3

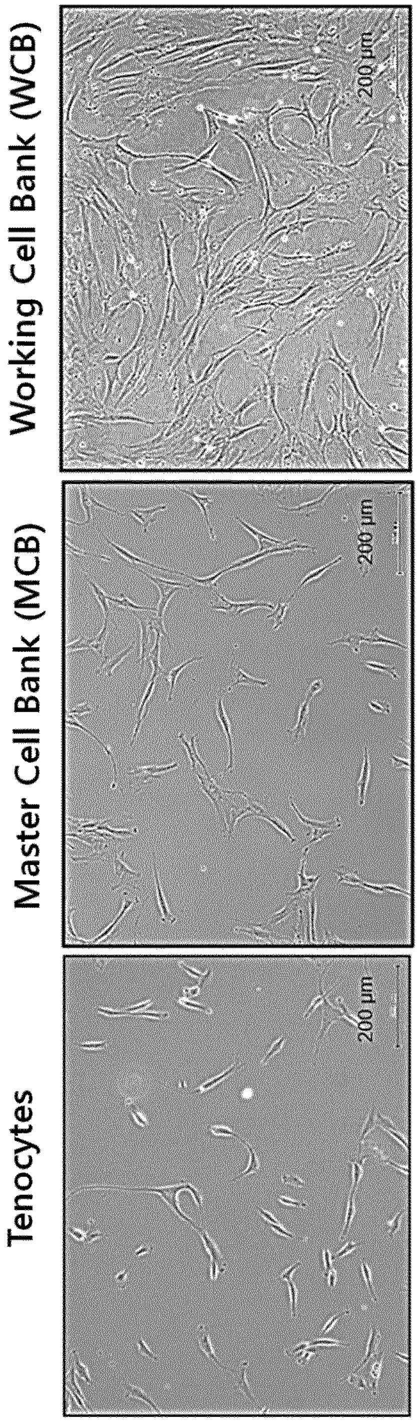
Working Cell Bank (WCB)
Master Cell Bank (MCB)
Tenocytes
200 µm
200 µm
200 µm

Fig 4

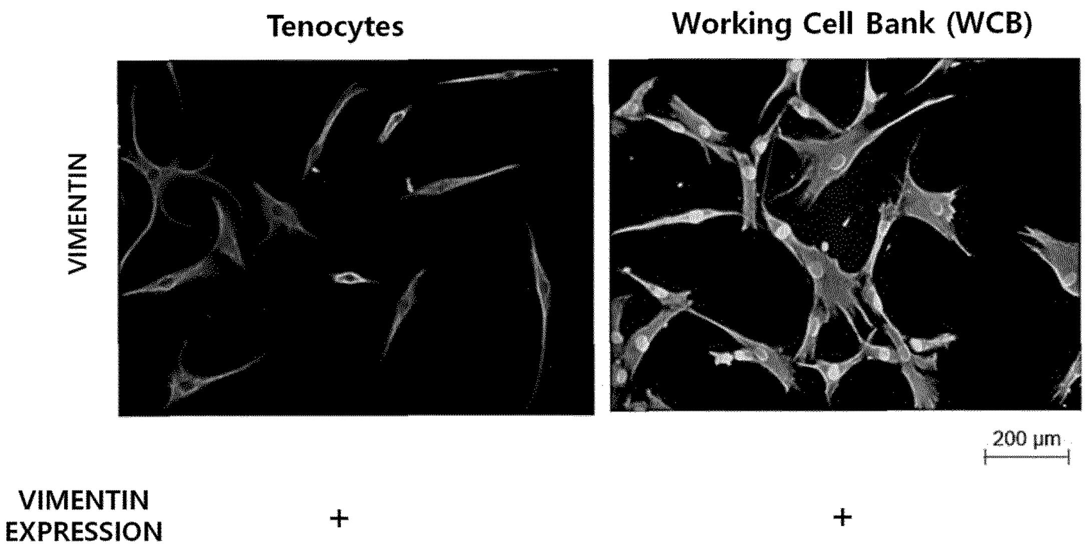
Tenocytes
Working Cell Bank (WCB)
VIMENTIN
200 μm
VIMENTIN EXPRESSION
+
+

Fig 5

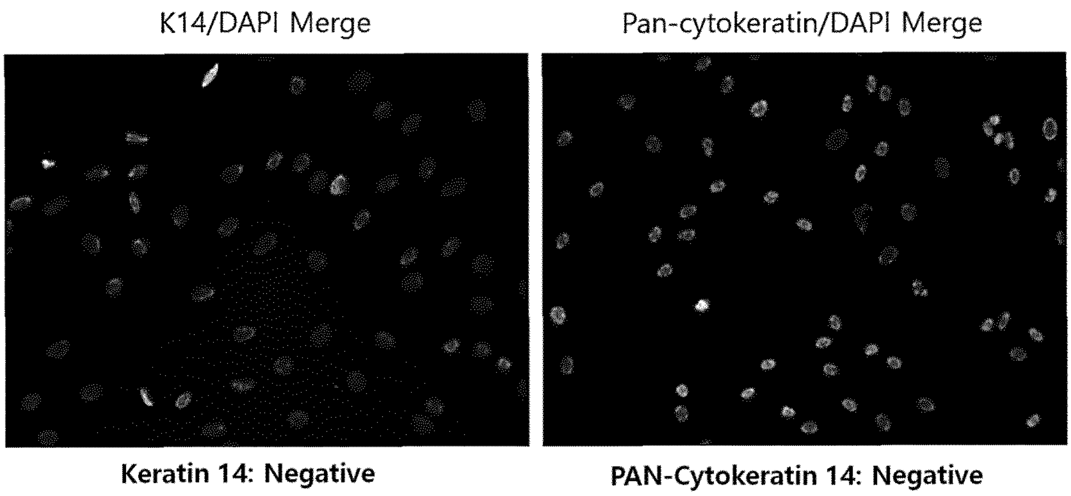
K14/DAPI Merge
Pan-cytokeratin/DAPI Merge
Keratin 14: Negative
PAN-Cytokeratin 14: Negative

Fig 6

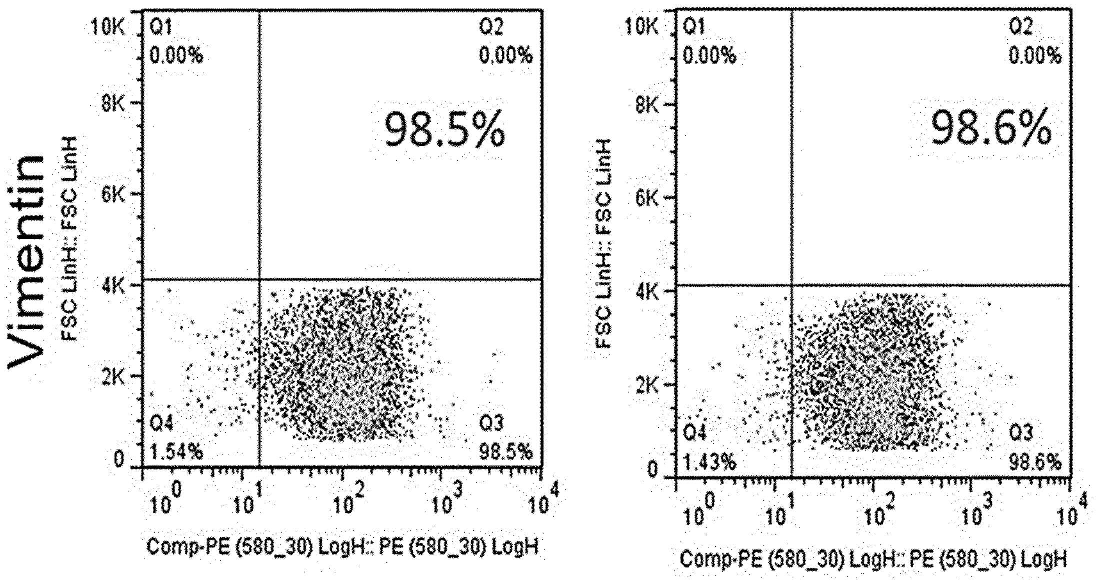
Vimentin
FSC LinH:: FSC LinH
10K
8K
6K
4K
2K
0
Q1
0.00%
Q2
0.00%
98.5%
Q4
1.54%
Q3
98.5%
$10^0$ $10^1$ $10^2$ $10^3$ $10^4$
Comp-PE (580_30) LogH:: PE (580_30) LogH
FSC LinH:: FSC LinH
Q1
0.00%
Q2
0.00%
98.6%
Q4
1.43%
Q3
98.6%
Comp-PE (580_30) LogH:: PE (580_30) LogH

Fig 7

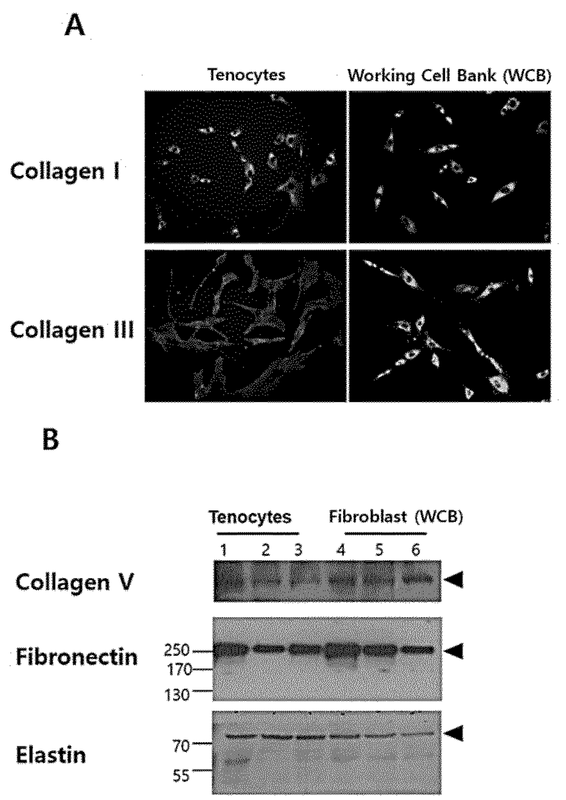
A
Tenocytes
Working Cell Bank (WCB)
Collagen I
Collagen III
B
Tenocytes
Fibroblast (WCB)
1 2 3 4 5 6
Collagen V
Fibronectin
250
170
130
Elastin
70
55

Fig 8

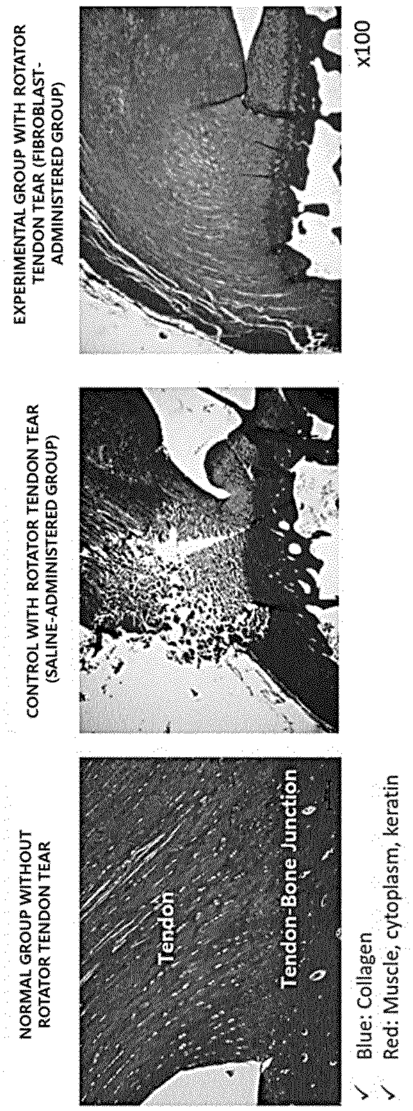
NORMAL GROUP WITHOUT ROTATOR TENDON TEAR
Tendon
Tendon-Bone Junction
CONTROL WITH ROTATOR TENDON TEAR (SALINE-ADMINISTERED GROUP)
EXPERIMENTAL GROUP WITH ROTATOR TENDON TEAR (FIBROBLAST-ADMINISTERED GROUP)
x100
✓ Blue: Collagen
✓ Red: Muscle, cytoplasm, keratin

Fig 9

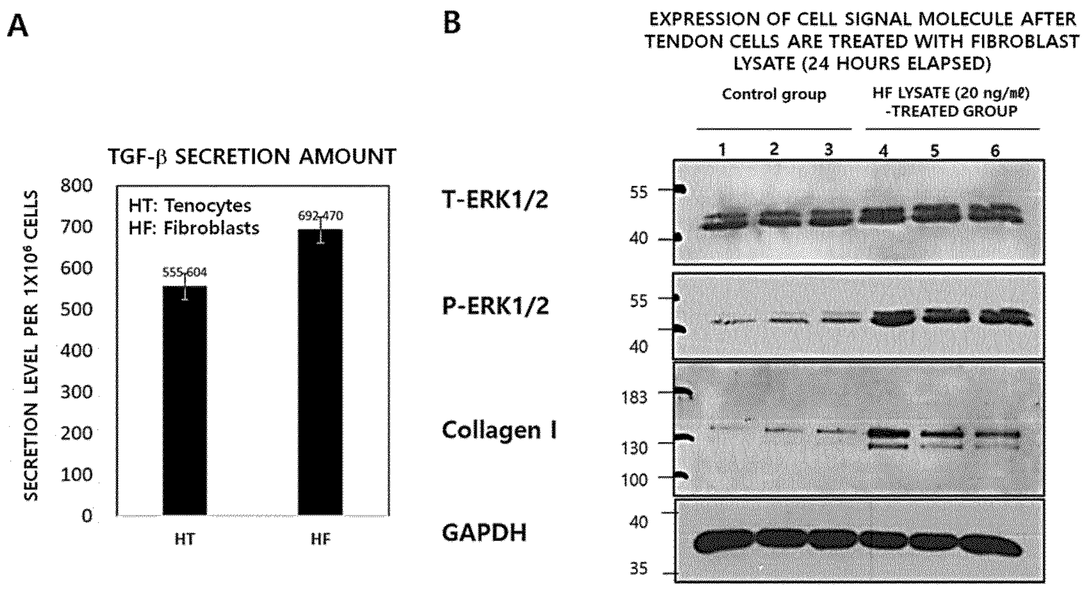
A
TGF-β SECRETION AMOUNT
SECRETION LEVEL PER 1X10⁶ CELLS
800
700
600
500
400
300
200
100
0
HT: Tenocytes
HF: Fibroblasts
555.604
692.470
HT
HF
B
EXPRESSION OF CELL SIGNAL MOLECULE AFTER TENDON CELLS ARE TREATED WITH FIBROBLAST LYSATE (24 HOURS ELAPSED)
Control group
HF LYSATE (20 ng/mℓ) -TREATED GROUP
1 2 3 4 5 6
T-ERK1/2
P-ERK1/2
Collagen I
GAPDH
55
40
55
40
183
130
100
40
35

Fig 10

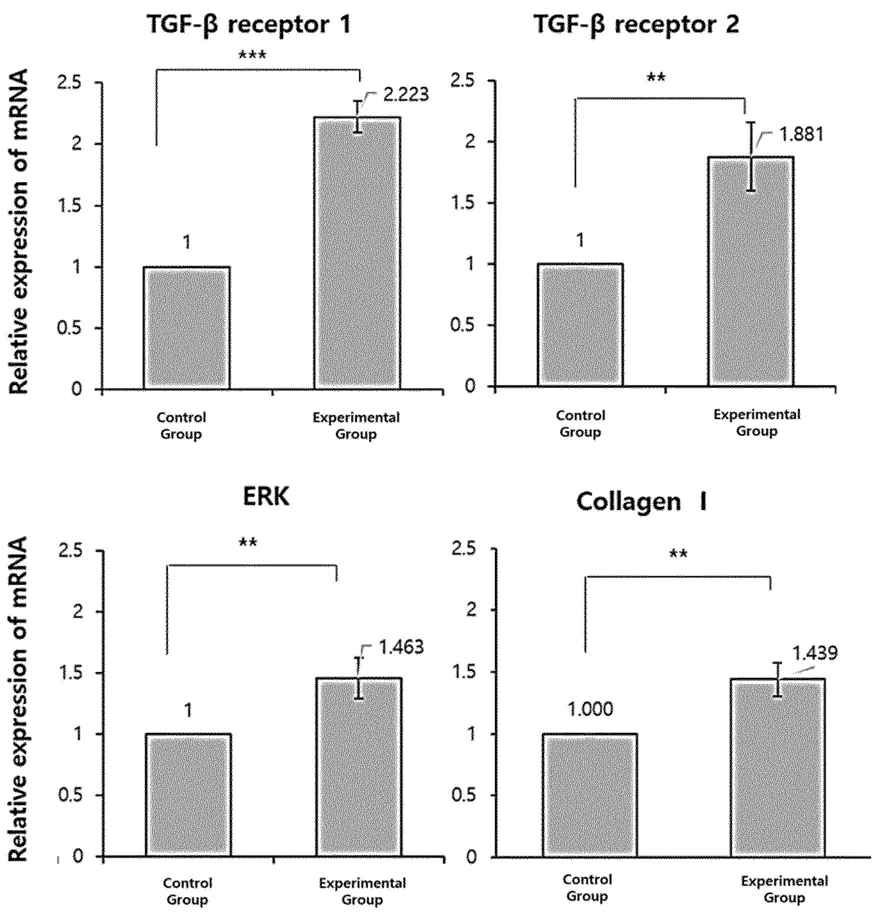
TGF-β receptor 1
TGF-β receptor 2
ERK
Collagen I
Relative expression of mRNA
***
**
2.223
1.881
1.463
1.439
1
1.000
2.5
2
1.5
1
0.5
0
Control Group
Experimental Group

## INTERNATIONAL SEARCH REPORT

International application No. **PCT/KR2021/005591**

**A. CLASSIFICATION OF SUBJECT MATTER**

**C12N 5/077**(2010.01)i; **C07K 14/78**(2006.01)i; **C07K 14/495**(2006.01)i; **A61K 35/33**(2014.01)i; **A61P 21/00**(2006.01)i

According to International Patent Classification (IPC) or to both national classification and IPC

**B. FIELDS SEARCHED**

Minimum documentation searched (classification system followed by classification symbols)

C12N 5/077(2010.01); A61K 47/36(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 건재생 (tendon generation), 피부유래섬유아세포 (dermal fibroblast), 비멘틴 (vimentin), 콜라겐 (collagen), TGF-β, 계대배양 (subculture)

**C. DOCUMENTS CONSIDERED TO BE RELEVANT**

| Category* | Citation of document, with indication, where appropriate, of the relevant passages | Relevant to claim No. |
|---|---|---|
| X<br>Y | KWON, J. et al. Effects of allogenic dermal fibroblasts on rotator cuff healing in a rabbit model of chronic tear. The American journal of sports medicine. 2018, vol. 46, no. 8, pp. 1901-1908.<br>See abstract; page 1902; page 1903; page 1906; page 1907; and figures 1-2. | 1-3,6-10<br>4-5 |
| Y | Retrieved from the Internet. Himedia CellCulture. EZXpandTM Dermal Fibroblast Culture Kit. January 2013. <ULR:https://himedialabs.com/TD/CCK027.pdf>.<br>See pages 3-4. | 4-5 |
| X | CHU, J. et al. Rebuilding Tendons: A Concise Review on the Potential of Dermal Fibroblasts. Cells. 08 September 2020, vol. 9, thesis no. 2047, pp. 1-24.<br>See abstract; page 8; page 12; page 16; page 17; page 19; and table 3. | 1,8-10 |
| A | JUHL, P. et al. Dermal fibroblasts have different extracellular matrix profiles induced by TGF-β, PDGF and IL-6 in a model for skin fibrosis. Scientific reports. 2020 [14 October 2020], vol. 10, no. 1, thesis no. 17300, pp. 1-10.<br>See entire document. | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 August 2021** | **17 August 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
**PCT/KR2021/005591**

**C. DOCUMENTS CONSIDERED TO BE RELEVANT**

| Category* | Citation of document, with indication, where appropriate, of the relevant passages | Relevant to claim No. |
|---|---|---|
| A | BHOGAL, R. K. et al. Regulatory effect of extracellular signal-regulated kinases (ERK) on type I collagen synthesis in human dermal fibroblasts stimulated by IL-4 and IL-13. International reviews of immunology. 2008, vol. 27, pp. 472-496. See entire document. | 1-10 |
| A | KR 10-2016-0105363 A (ANTEROGEN CO., LTD.) 06 September 2016 (2016-09-06) See entire document. | 1-10 |

Form PCT/ISA/210 (second sheet) (July 2019)

**INTERNATIONAL SEARCH REPORT**

International application No.
**PCT/KR2021/005591**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11**
because they relate to subject matter not required to be searched by this Authority, namely:

Claim 11 pertains to a method for treatment of the human body, and thus pertains to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2021/005591**

| Patent document cited in search report | Publication date (day/month/year) | Patent family member(s) | Publication date (day/month/year) |
|---|---|---|---|
| KR 10-2016-0105363 A | 06 September 2016 | KR 10-2015-0138700 A | 10 December 2015 |

Form PCT/ISA/210 (patent family annex) (July 2019)